# FASCICULE DE BREVET EUROPEEN

(11) **EP 0 643 582 B1**
(45) Date de publication et mention de la délivrance du brevet: **12.08.1998**
(21) Numéro de dépôt: 93913100.9
(22) Date de dépôt: 07.06.1993
(51) Int. Cl.: A61K 35/14, A61M 1/36

(54) **PROCEDE D'OBTENTION D'UN SURNAGEANT DE THROMBOCYTES ACTIVES**
VERFAHREN ZUR ERHALTUNG EINER UEBERSTANDSFRAKTION VON AKTIVIERTEN THROMBOZYTEN
METHOD FOR PRODUCING A SUPERNATANT OF ACTIVATED THROMBOCYTES

(30) Priorité: 05.06.1992 FR 9206826
(43) Date de publication de la demande: 22.03.1995
(73) Titulaire: INOTEB, F-56920 Saint-Gonnery (FR)
(72) Inventeur: DELMAS, Olivier, Marcel, Joseph, F-91310 Montlhery (FR)
(74) Mandataire: Tonnellier, Jean-Claude
(86) Numéro de dépôt international: FR9300544
(87) Numéro de publication internationale: WO9325215

(56) Documents cités:
- EP-A- 0 349 188
- WO-A-86/07279
- WO-A-90/07931
- FR-A- 2 513 896
- US-A- 4 360 435
- US-A- 4 410 630
- US-A- 4 618 494

## Description

La présente invention concerne un procédé de préparation de facteurs plaquettaires (contenus des granules thrombocytaires) et un dispositif permettant l'activation des thrombocytes, d'origine humaine ou animale, emmagasinés sur ou dans une trame polymérique en vue de l'obtention en un temps court, et sans risque de contamination extérieure, de molécules solubles possédant une activité biologique.

La solution de facteurs plaquettaires libérés par les thrombocytes (appelés aussi "plaquettes") activés est souvent désignée par l'expression "surnageant de thrombocytes activés".

Aujourd'hui, les surnageants de thrombocytes activés sont utilisés comme matière première pour la purification de molécules biologiques, comme par exemple : le "platelet-derived growth factor", le "Transforming growth factor β", le "Basic fibroblast growth factor", le "Platelet factor 4", le "platelet-derived endothelial growth factor", l'"heparin-binding epidermal growth factor", le "Insulin-like growth factor 1", le "connective tissue activating peptide III", la β-thromboglobuline, l'"epidermal growth factor", le plasminogène, le facteur Von Willebrand, le fibrinogène, la sérotonine, l'histamine, l'adénosine di- et triphosphate, la fibronectine, la vitronectine, le facteur XIII, des enzymes protéolytiques ou glycolytiques, les métabolites de l'acide arachidonique (voir pour revue, Inflammation and Repair, H.L. Wong & S.M. Wahl, In "Peptide Growth Factors and their Receptors" p 510, Sporn & Roberts Eds, Springer-Verlag, Berlin; Platelets and Response to Injury, R.A. Terkeltaub & M.H. Ginsberg, In "The Molecular and cellular biology of wound repair" p 38, Clark & Henson Eds, Plenum Press, New-York).

Certaines méthodes de purification sont connues et ont pu faire l'objet de dépôts de brevets. On peut citer notamment : demande EP-A-0 323 842 concernant la purification du "transforming growth factor β", Brevet U.S. n° 4,479,896 concernant la purification du "platelet-derived growth factor".

Les surnageants de thrombocytes activés (ou une fraction de ces surnageants) peuvent-être utilisés en thérapeutique, par exemple pour leur activité cicatrisante (D. Knighton et al., Ann Surg (1982)196 379-388; D.M. Carter et al., In : Growth factors and other aspects in Wound Healing, p 303-317, Barbul, Pines, Cadwell, Hunt Eds, Alan R. Liss Inc. New-York 1988; brevet U.S. n° 4,760,131; brevet WO 88/03409; brevet WO 86/03122; brevet WO 89/05656), ou en cosmétologie, par exemple pour leur effet bénéfique pour lutter contre l'alopécie (brevet WO 90/07931).

Les facteurs plaquettaires contenus dans les surnageants de thrombocytes activés peuvent être utilisés notamment dans le traitement d'ulcères.

Les méthodes décrites jusqu'à présent pour la préparation de surnageants thrombocytaires sont longues (durée minimum d'une heure 30 minutes environ), fastidieuses (car nécessitant des transferts de tube à tube, des équilibrages de centrifugation, des pipetages) et pratiquement impossibles à automatiser, et elles présentent des risques de contamination du produit par son environnement (transferts de liquides à col ouvert), ou du manipulateur par le produit manipulé, lequel est potentiellement contaminé par des germes transmissibles

En effet, ces méthodes de préparation début par l'isolement des thrombocytes à partir du sang par centrifugations successives. Ceux-ci sont alors lavés par centrifugation pour éliminer le plasma (Isolation of platelets, In: Methods in enzymology, Section I, pp 3-27, Vol. 169 Hawiger Ed, Academic Press London 1989; demande de brevet WO 86/03122, brevet U.S. n° 4,760,131). Les thrombocytes purifiés sont alors mis en suspension en présence d'un activateur. De nombreux activateurs sont connus (Platelets and Response to Injury, R.A. Terkeltaub & M.H. Ginsberg, In "The Molecular and cellular biology of wound repair" pp 35-55 , Clark & Henson Eds, Plenum Press, New-York). On distingue trois types d'activateurs : les activateurs forts qui sont capable d'induire la sécrétion de tous les granules (thrombine, collagène, l'ionophore de calcium A23187), les activateurs intermédiaires comme le thromboxane A2, l'ADP en présence ou non d'ions calcium, l'adrénaline, et les activateurs faibles n'induisant pas la sécrétion granulaire (sérotonine). L'activateur le plus utilisé est la thrombine. D'autres procédés d'activation utilisent des méthodes physiques de lyse des thrombocytes par congélations-décongélations successives ou par les ultra-sons. Le surnageant thrombocytaire est clarifié par centrifugation et/ou filtration afin d'éliminer les membranes thrombocytaires et autres éléments insolubles.

En outre, dans les techniques classiques, les contaminants d'origine plasmatique sont difficiles à éliminer, et les lavages successifs visant à éliminer ces contaminants plasmatiques entraînent une diminution du rendement en molécules thrombocytaires par perte des thrombocytes ou par activation partielle de ceux-ci. Enfin, le plasma dans lequel les thrombocytes sont en suspension est difficilement récupérable pour un autre usage.

La présente invention permet de résoudre les problèmes exposés et inhérents aux méthodes actuelles de préparation de surnageants plaquettaires, tels que les risques de contamination, la perte de rendement, la nécessité de manipulations longues et délicates.

L'invention a pour objet un procédé d'obtention d'une solution de facteurs plaquettaires comportant une étape d'activation de thrombocytes par mise en contact avec une solution d'activateur de thrombocytes, et dans lequel on recueille une solution des facteurs plaquettaires libérés par les thrombocytes lors de l'étape d'activation, caractérisé par le fait que l'on fait passer un liquide contenant une suspension de thrombocytes sur un filtre capable de retenir les thrombocytes, que l'on ajoute une solution d'activateur aux thrombocytes retenus sur le filtre, et que l'on sépare par filtration un filtrat contenant les facteurs plaquettaires en solution tandis que les thrombocytes restent retenus sur le filtre.

Il convient de remarquer qu'il n'était pas évident de chercher à maîtriser l'activation des thrombocytes en opérant sur un filtre, car on devait normalement s'attendre à une activation spontanée des thrombocytes sur le filtre ; voir par exemple J.N. LINDON et al., Méthods in Enzymology, 169, 104-107 (1989).

Effectivement, l'art antérieur ne suggérait pas de remplacer les méthodes classiques de centrifugation par une simple filtration, et on peut estimer que cela est en relation avec l'existence d'un préjugé contre la filtration, dont on redoutait qu'elle provoque une activation précoce, spontanée, des thrombocytes, empêchant la purification des facteurs plaquettaires, c'est-à-dire leur séparation d'avec une proportion importante des constituants plasmatiques. En effet, en cas d'activation spontanée, les facteurs plaquettaires produits, qui sont solubles, restent avec les autres produits plasmatiques passant à travers le filtre, ou passent à travers le filtre avec les eaux de lavage éventuellement utilisées dans un but de purification avant l'étape d'activation déclenchée par un activateur, et dans ce cas, cette étape d'activation serait inopérante car trop tardive, ou ne permettrait d'obtenir les facteurs plaquettaires qu'avec un très faible rendement.

On a maintenant découvert qu'en fait, de façon surprenante, il est possible, avec de nombreux filtres connus, y compris des filtres commerciaux, de retenir sur filtre les thrombocytes sans observer une activation spontanée susceptible d'entraîner des pertes importantes en facteurs plaquettaires. Les filtres qui conviennent peuvent être déterminés par de simples expériences de routine. On a découvert qu'il est même possible, après avoir retenu les thrombocytes sur un filtre, de procéder à un lavage préliminaire, avant de provoquer l'activation à l'aide d'un activateur, sans perte rédhibitoire de facteurs plaquettaires. On peut donc ainsi obtenir des préparations de facteurs plaquettaires purifiés, ne contenant que de faibles proportions de constituants plasmatiques.

L' invention a également pour objet un dispositif permettant de mettre en oeuvre, de façon simple, le procédé qui vient d'être décrit. Ce dispositif, qui se présente sous forme d'un ensemble clos permettant l'activation de thrombocytes et l'obtention, de façon stérile, d'une solution de facteurs plaquettaires au départ d'un liquide contenant une suspension de thrombocytes est caractérisé par le fait qu'il comprend des moyens de filtration contenant un filtre capable de retenir les thrombocytes, le dit filtre étant placé dans une enceinte et délimitant dans la dite enceinte une partie amont et une partie aval, la partie amont de la dite enceinte étant en communication avec au moins un conduit, muni de moyens d'ouverture et de fermeture appropriés, raccordant ou permettant de raccorder la partie amont à un réservoir contenant le liquide de départ, et la partie aval de ladite enceinte étant en communication avec au moins un conduit, muni de moyens d'ouverture et de fermeture appropriés, permettant de recueillir successivement le filtrat du liquide de départ et la dite solution de facteurs plaquettaires, et par le fait qu'il comprend en outre un réservoir contenant un activateur de thrombocyte, le dit réservoir étant raccordé ou raccordable avec la partie amont de façon à permettre l'introduction dans la dite partie amont de l'activateur sous la forme d'une solution.

Le dispositif de l'invention se présente donc de préférence, sous la forme d'un kit prêt à l'emploi, contenant l'activateur, soit sous forme de solution dans un réservoir approprié, soit sous forme d'une poudre, notamment dans le cas de la thrombine. Dans ce dernier cas, on reconstitue la solution d'activateur par addition à la poudre d'un véhicule liquide approprié. Le véhicule liquide peut être un véhicule stérile contenu dans une poche satellite. On peut également reconstituer la solution d' activateur avec un véhicule liquide usuel ou la préparer extemporanément, la stérilisation étant assurée en munissant la tubulure d'acheminement de la solution d'activateur d'un filtre stérilisant. On peut procéder de même pour la solution de lavage.

Plus particulièrement, la présente invention concerne un dispositif permettant l'obtention d'un surnageant (12) de thrombocytes activés, caractérisé en ce qu'il est constitué d'un filtre (5) de nature à retenir les thrombocytes, ledit filtre (5) étant relié d'une part, en amont à un premier moyen d'acheminement (2) des thrombocytes, en suspension dans un liquide (15), et à un second moyen d'acheminement (4) d'une solution d'activateurs de thrombocytes (7) et d'autre part, en aval à un moyen d'évacuation (8) dudit liquide de suspension (13) et à un moyen de récupération (9) dudit surnageant (12), lesdits moyens étant obturables réversiblement par des moyens appropriés (6).

Le dispositif est de préférence complété grâce à un ensemble par lequel il est relié en amont à un moyen d'acheminement d'un liquide de lavage (14) notamment un tampon de lavage pouvant contenir un adjuvant et en aval à un moyen d'évacuation (10) du liquide de lavage usagé (16).

Dans ce dispositif, lesdits moyens d'acheminement des thrombocytes en suspension dans un liquide et de la solution d'activateurs de thrombocytes et éventuellement du liquide de lavage, sont des tubulures reliées chacune à un réservoir destiné à contenir respectivement ladite suspension de thrombocytes, ladite solution d'activateurs de thrombocytes et ledit liquide de lavage, lesdits moyens d'évacuation étant chacun une tubulure reliée à un récipient destiné à contenir respectivement le liquide de suspension des thrombocytes après filtration et le liquide de lavage, ledit moyen de récupération étant une tubulure reliée à un récipient destiné à contenir le surnageant de thrombocytes activés.

Le dispositif selon la présente invention étant généralement destiné à une utilisation unique, on utilisera de préférence des réservoirs et des récipients qui sont constitués chacun d'une ou plusieurs poches de transfert simple éventuellement juxtaposées, ou des seringues elles aussi jetables. Les moyens d'obturation sont des serre-fils s'appuyant sur une tubulure en plastique déformable de qualité médicale, par exemple des polyéthylènes.

Ces dispositifs peuvent être complétés par des moyens de dérivation du filtre pour éviter les contaminations du filtre par le contenu des poches et le moyen de récupération du surnageant est relié à un moyen destiné à diminuer la charge virale du surnageant.

Les différents liquides sont acheminés de préférence par gravité ou par des systèmes de pompe de type péristaltique par exemple, et l'ensemble est clos et stérile.

Comme cela est indiqué, la suspension liquide contenant les thrombocytes est de préférence du sang total d'origine humaine ou animale ou l'une des fractions du sang renfermant des thrombocytes, notamment un plasma ou un plasma riche en plaquettes.

La solution d'activateurs de thrombocytes est de préférence une solution aqueuse.

Le dispositif selon la présente invention permet d'obtenir des surnageants de thrombocytes activés dans des conditions de stérilité parfaite et à des coûts compatibles avec une application autologue.

D'autres caractéristiques et avantages des procédé et dispositif selon l'invention seront mis en évidence à la lecture de la description détaillée ci-après, faisant référence aux dessins annexés dans lesquels :
- la figure 1 représente de façon schématique un mode de réalisation particulier du dispositif de l'invention
- et la figure 2 représente de façon schématique un mode de réalisation particulier, décrit à l'exemple 6 ci-après, du système filtrant.

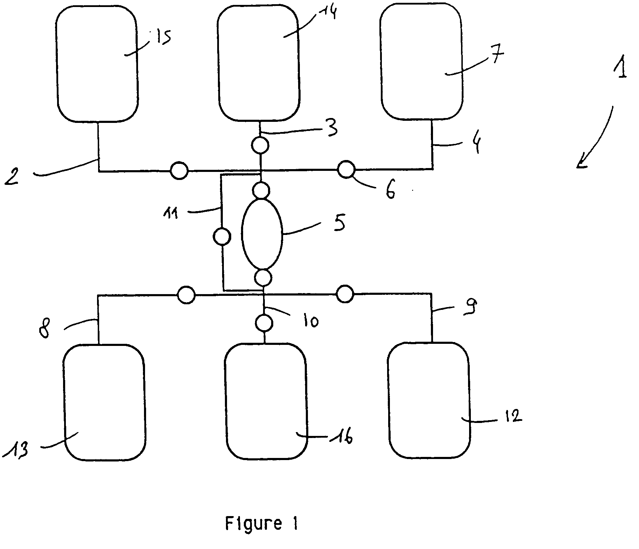

Le dispositif (1) de la présente invention permet l'obtention d'un surnageant de thrombocytes activés comme celui décrit dans la figure 1.

Un filtre (5) destiné à retenir les thrombocytes constitue la partie centrale du dispositif. Le système de filtration peut-être constitué par exemple par un filtre en profondeur ou par un filtre microporeux. La filtration peut-être frontale ou tangentielle.

Un système de filtration en profondeur peut être par exemple constitué d'un filtre en polyester non tissé, destiné à déleucocyter les concentrés globulaires (Filtres Erypur Optima G-0 et G-2 de la Société Organon-Teknika, Fresnes, France ; filtres Pall RC100 et RC50, Pall Biomedical, Paris ; Filtres Sepacell R500 Asahi Medical, Frankfurt/Main, Allemagne). En fonction de leurs caractéristiques et de leur usage, ces systèmes retiennent de 50% à 100% des thrombocytes. D'autres media filtrants conçus particulièrement pour la rétention des thrombocytes sont connus (EP-A-0 315 022)

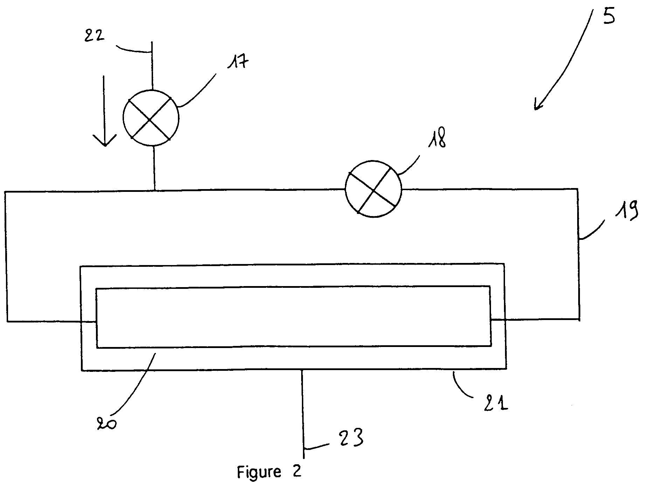

Un système de filtration microporeux peut être, par exemple une cartouche (21) de fibres creuses (20) (cartouches A/G Technology, Microgon, Gambro, Enka, demande EP-A-0 116 626) comme décrit dans la figure 2. La filtration sur media microporeux est réalisée par exemple de manière tangentielle. Afin de réaliser une filtration tangentielle, une tubulure (19) est connectée aux deux extrémités des fibres (20). Une pompe, par exemple peristaltique (18), associée à cette tubulure assure la circulation du rétentat dans les fibres filtrantes. Une tubulure (22) munie d'une pompe, par exemple péristaltique (17), et reliée aux tubulures amont et à (19) permet d'acheminer vers (19) les solutions à filtrer. Une tubulure (23) connectée à la cartouche (21) constitue la sortie du filtre.

Ce filtre (5) est relié en amont par un moyen d'acheminement (2) à un récipient contenant un liquide biologique (15) renfermant des thrombocytes en suspension, lequel peut être du plasma riche en plaquettes, du buffy-coat, des concentrés plaquettaires standards, ou des concentrés plaquettaires unitaires.

Le filtre (5) est également relié en amont par un moyen d'acheminement (3) à un récipient contenant une solution d'activateurs de thrombocytes (7). L'activateur peut être de la thrombine, de l'adénosine di-phosphate, du collagène, l'ionophore du calcium A 23187.

En outre, le filtre (5) est relié en aval par un moyen d'évacuation (8) à un récipient qui reçoit le liquide biologique (13) qui contenait les thrombocytes en suspension. Il est également relié par le moyen de récupération (9) à un récipient qui reçoit le surnageant de thrombocytes activés (12).

A ce dispositif, on peut ajouter un moyen d'acheminement (3) d'un liquide de lavage (14) des thrombocytes, le liquide usagé (16) étant évacué par le moyen d'évacuation (10) dans un récipient destiné à le recevoir.

Les réservoirs et récipients contenant (15), (14), (7), (8), (13), (16), (12) sont par exemple des poches de transfert telles que les poches de transfert souples couramment utilisées dans la collecte de produits sanguins. Chacun des moyens d'acheminement et d'évacuation peut être une tubulure. Chaque tubulure peut être obturée à l'aide d'un serre-fil par exemple.

L'ensemble des composants de ce dispositif forme un circuit clos (poches, tubulures, aiguilles, raccords, filtres, solutions) pouvant être stérilisé avant utilisation par des moyens appropriés.

Un des modes d'emploi du dispositif est le suivant :

Les tubulures (3), (4), (9) et (10) sont obturées. Le conteneur contenant les thrombocytes en suspension (15) est vidé par gravité (ou par un système de pompage ayant pour principe l'écrasement de la tubulure ou de la poche à vider ou la mise en oeuvre de pistons de seringues stériles) à travers le filtre (5) dans la poche de recueil (13). Les tubulures (2) et (8) sont obturées. Les tubulures (3) et (10) sont libérées. La solution de lavage (14) est évacué par gravité après passage sur le filtre (5) dans la poche destinée à recevoir la solution de lavage usagée (16). Les tubulures (3) et (10) sont obtures et les tubulures (4) et (9) sont libérées. La poche (7) est vidée gravité et le surnageant obtenu (12) est recueilli dans une poche. Toutes les tubulures sont alors fermées.

Dans le cas de l'utilisàtion d'un système de filtration tangentielle, une pompe péristaltique (17) achemine (15), (14) ou (7) vers la tubulure (19). Une pompe péristaltique (18) sur (19) assure la recirculation du rétentat dans les fibres filtrantes. Les pompes péristaltiques sont en mouvement pendant toute la durée des opérations décrites au paragraphe précédent.

Certaines variantes de ce dispositif sont possibles. On peut citer à titre d' exemples les variantes suivantes :
a) Une tubulure de dérivation du filtre incluant un dispositif d'obstruction (11) peut être ajoutée afin de purger le filtre ou pour éviter l'écoulement sur le filtre d'une partie de (15), (14) ou (7). Par exemple, du sang total peut être centrifugé dans une seringue, l'orifice de la seringue branché à la place de la poche contenant (15), les érythrocytes sédimentés dans la seringue poussés dans la dérivation, et le plasma surnageant poussé à travers le filtre.
b) Le recueil de (16) peut être réalisée dans la poche de recueil de (13).
c) La poche de recueil du surnageant (12) peut contenir un excipient de quelque nature que ce soit, afin soit d'enrichir le surnageant plaquettaire, par exemple par des adjuvants de la cicatrisation ou des stabilisants ou afin de diluer le surnageant dans des proportions données.
d) Chacune des poches peut être munie de dispositif divers, comme des sites de prélèvement.
e) La poche de récupération du surnageant peut (12) peut être complétée de prises de prélèvement ou bien connectée à des poches plus petites de façon à permettre en circuit clos une répartition, de manière compatible à une utilisation du produit lors d'un traitement au long cours. Des raccords de tubulure peuvent être créés de façon stérile au moyen de dispositifs de soudures connus pour réaliser toute conception de montages stériles adaptés aux opérations de mélange, séparation, répartition.
f) Le surnageant (12) pourra subir une étape d'inactivation virale, soit par pasteurisation par immersion dans un bain-marie dans des conditions validées, soit par des anticorps neutralisant, soit par filtration (système Virosolve développé par Millipore, Bedford, USA; système Asahi; système Pall), soit par tout autre procédé efficace par diminuer la charge virale tout en conservant une activité suffisante au produit traité. Le système d'inactivation virale peut-être réalisé de manière à être intégré dans le dispositif précédemment décrit, et par conséquent conserver le caractère clos dudit dispositif.
g) Une matrice retenant spécifiquement certaines molécules du surnageant thrombocytaire peut être montée en amont ou en aval de la poche de recueil du surnageant (12), afin d'effectuer une étape directe de purification de molécules biologiques.

De manière générale tout dispositif peut-être ajouté dans la mesure où il conserve le caractère clos de l'ensemble.
La présente invention est illustrée par les exemples suivants où les chiffres de (1) à (16) font référence à la figure 1 et les chiffres de (17) à (23) font référence à la figure 2.

### Exemple 1

a- Une poche de transfert de 400 ml (Baxter référence R2074) est remplie stérilement par 350 ml d'une solution de Tris 0,04 M, HCl, pH 7,4, NaCl 0,15 M (14).
b- Une poche identique est remplie par 250 ml de la même solution et ènrichie par 1250 U-NIH de thrombine bovine (Roche, référence 07 2846 2) (7).
c- Aprés avoir clampé toutes les tubulures, un filtre Optima G2 (Organon Teknika, Fresnes, France) est connectée sous atmosphère stérile par son extrémité amont rouge à un mélange de cinq concentrés plaquettaires standards (15) contenu dans une poche de transfert de même type qu'en a-.
d- L'extrémité amont blanche du filtre est connectée à la poche contenant (7). Par fabrication, le filtre comporte en aval deux poches de transfert numérotés 1 et 2 utilisées pour recueillir respectivement l'une (12), l'autre (13) et (16).
e- La poche contenant (15) est surélevée de 5 à 50 cm, et la poche 2 est abaissée par rapport au filtre de la même hauteur.
f- Les tubulures (2) et (8) sont libérées. Le plasma (15) s'écoule dans la poche (2) en 10 minutes plus ou moins 5 minutes.
g- Les tubulures sont à nouveau obturées.
h- Sous atmosphère stérile, la poche ayant contenu (15) est retirée. La poche contenant (14) est connectée à la place de la poche ayant contenu (15). On répète les opérations comme décrit en e, f et g.
i- Les tubulures (4) et (9) sont libérées et l'on répète les opérations comme décrit en e, f et g.
j- La tubulure (9) est alors fermée par soudure et coupée.
(15) renferme au départ 318.10⁹ thrombocytes dans 243 ml de plasma. Après la manipulation, (15) et (16) renferment 13,65.10⁹ thrombocytes. La filtre a par conséquent retenu 95,7% des thrombocytes.
Les résultats des analyses effectuées sur (12) sont consignés dans le tableau I.

Ou peut remplacer la thrombine par une solution équivalente d'ADP.

### Exemple 2

Le dispositif est préparé et utilisé comme dans l'exemple 1, avec les modifications suivantes: (14) a un volume de 400 ml de tampon, la poche contenant (15) est une poche de transfert de 800 ml (Fenwal réf. R053) remplie par un mélange de trois plasmas riches en plaquettes. Le contenu de la poche 2 (13) est transféré en continu durant la filtration des plasmas dans une poche annexe de 800 ml connectée à la poche 2. Après la filtration, (14) est vidée à travers le filtre dans la poche 2.
(15) renferme au départ 198,6.10⁹ thrombocytes dans 782 ml de plasma. Après la manipulation, (13) et (16) renferment ensemble 14,4.10⁹ thrombocytes. Le filtre a par conséquent retenu 96,5% des thrombocytes.
Les résultats des analyses effectuées sur (12) sont consignés dans le tableau I.

### Exemple 3

Le dispositif est préparé et utilisé comme dans l'exemple 1, avec les modifications suivantes: (14) a un volume de 360 ml de tampon, la poche contenant (15) renferme un mélange de trois concentrés leuco-plaquettaires (ou buffy-coat) (15).
(15) renferme au départ 125.10⁹ thrombocytes. Après la manipulation, La poche 2 contenant (13) et (16) renferme 4,8.10⁹ thrombocytes. Le filtre a par conséquent retenu 96,16% des thrombocytes.
Les résultats des analyses effectuées (12) sur sont consignés dans le tableau I.

Cet exemple montre qu'avec le procédé de l'invention, il est possible d'éliminer une forte proportion des protéines plasmatiques, même au départ d'un concentré leuco-plaquettaire. On rappelle qu'un plasma contient au moins 0,3g environ de protéines libres par milliard de plaquettes.

### Exemple 4

Le dispositif est préparé et utilisé comme dans l'exemple 1 avec les modifications suivantes : la poche contenant (14) renferme 464 ml d'un tampon Hepes (12 g/l), NaCl (5,8 g/l), Glucose (5,4 g/l), KCl (0,25 g/l) (14), la poche contenant (7) renferme 221 ml de ce même tampon enrichi par 221 U de thrombine humaine (7).
La durée totale de la manipulation est de 36 minutes, dont 7 minutes pour la filtration des concentrés plaquettaires standards, 14 minutes pour le lavage du filtre (écoulement de 14) et 10 minutes pour l'activation des thrombocytes (écoulement de (7) à travers le filtre (5)). 98% des thrombocytes sont retenus sur le filtre.
Les résultats des analyses effectuées sur (12) sont consignés dans le tableau I.

### Exemple 5

On réalise le montage suivant : un filtre Sepacell R-500B1 (ASAHI) est connecté en amont et en aval, à l'aide d'un appareil SCD IIB (DU PONT), à une tubulure triple munie d'obturateurs et de perforateurs. Six poches de 400 ml (Baxter réf. R 2074) sont montées une à une sur chaque perforateur de manière à réaliser le dispositif décrit dans la figure unique. Les poches placées en aval et destinées à recueillir (12), (13) et (16) sont vides. (15) contenu dans l'une des poches de transfert amont, est un mélange de cinq concentrés standards de plaquettes. (14), contenu dans la deuxième poche de transfert amont est constitué de 447 ml du tampon Hepes décrit dans l'ensemble 4. (7), contenu dans la troisième poche de transfert amont, est constitué de 245 ml du même tampon enrichi par 245 U de thrombine humaine.
Les opérations de filtration, lavage et activation sont réalisée de manière semblable aux opérations décrites dans l'exemple 1.
Compte tenu que l'ensemble du dispositif est entièrement clos, la manipulation est réalisée entièrement hors atmosphère stérile.
La durée totale de la manipulation est de 41 minutes (dont 7 minutes pour la filtration, 11 minutes pour le lavage et 12 minutes pour l'activation).
Le filtre a retenu 89% des thrombocytes.
(13) renferme 175 ml de plasma pauvre en plaquettes, soit 87,5% du volume total de plasma filtré.
Les résultats des analyses effectuées sur (12) sont consignés dans le tableau I.

### Exemple 6

On réaise le montage suivant : une cartouche de fibres creuses PF 2000 (GAMBRO) est connectée à des tubulures de manière à réaliser le système filtrant décrit dans la figure 2. (22) est relié aux tubulures (2), (3) et (4). (23) est relié aux tubulures (8), (9) et (10) de manière à réaliser le dispositif décrit dans la figure 1. (15), contenu dans l'une des poches de transfert amont, est un mélange de trois plasmas riches en plaquettes. (14), contenu dans la deuxième poche de transfert amont est constitué de 740 ml du tampon Hepes décrit dans l'exemple 4. Cette deuxième poche amont renferme en outre environ 200 ml d'air stérile afin de purger le système à la fin de l'opération de lavage. (7), contenu dans la troisième poche de transfert amont, est constitué de 450 ml du même tampon enrichi par 2000 U de thrombine humaine. La pompe péristaltique (17) assure un débit continu de 22 ml/min et la pompe péristaltique (18) assure un débit continu de 80 ml/min.
Les opérations de filtration, lavage et activation sont réalisées de manière semblable aux opérations décrites dans l'exemple 1.
Compte tenu que l'ensemble du dispositif est entièrement clos, la manipulation est réalisée entièrement hors atmosphère stérile.
A l'issue de la manipulation aucune plaquette n'a pu être détectée dans (12), (13) et (16) Le filtre a donc retenu la totalité des plaquettes.
Les résultats des analyses effectuées sur (12) sont consignés dans le tableau I.

### Exemple 7 :

On utilise une cartouche de fibres creuses (A/G TECHNOLOGY) : porosité de 0,2µm ; Diamètre interne des fibres : 0,75 mm ; surface totale de filtration : 0,009m². Un tel filtre permet une filtration stérilisante, car il retient les bactéries. Le montage est réalisé comme indiqué dans la figure 2, sauf que la pompe (17) est omise. Le débit de la pompe 18 est de 130 ml/min.
Le produit de départ est un plasma riche en plaquettes de volume 195 ml. Les autres produits utilisés sont les mêmes que ceux décrits à l'exemple 6.
On opère de façon analogue à celle décrite précédemment à l'exemple 6.
Résultats, par milliard de thrombocytes : β thromboglobuline 50,3 µg
Volume de l'extrait plaquettaire recueilli : 156 ml
Volume de la solution de lavage usagée : 184 ml

La solution de lavage renferme moins de 1% (0,89%) de la quantité totale de β thromboglobuline contenue dans l'extrait plaquettaire.
Cela signifie qu'il n'y a pas eu d'activation précoce des thrombocytes au cours de l'opération de lavage.
On peut remplacer la thrombine par de l'ADP.

### Avantages de la présente invention par rapport aux méthodes classiques

Les résultats présentés dans les exemples permettent de dégager les avantages suivants:
Gain de temps : durée totale de 30 à 45 minutes à partir de l'obtention du plasma riche en thrombocytes, au lieu d'un minimum de 60 minutes avec les méthodes classiques.
Stérilité : le produit d'activation des thrombocytes est obtenu en circuit clos, éliminant les risques de contaminations provenant de l'extérieur.
Protection du manipulateur : le sang ou ses dérivés sont potentiellement dangereux. Le manipulateur ne peut en aucun cas être en contact direct avec les produits sanguins.
Facilité d'utilisation : le nombre d'opérations est considérablement réduit. Les opérations se rédnisent à des manipulations simples de transferts de liquides en circuit clos.
Investissement en matériel minimum : les enceintes protégées ou stériles ne sont pas requises (économies de hottes, boîte à gants, pièce classe 10,000), économie d'une centrifugeuse, de tubes et matériel à usage unique divers.
Produit final de meilleure qualité : les thrombocytes sont lavés bien plus abondamment, par comparaison avec des lavages par centrifugations successives. Par conséquent les protéines plasmatiques sont présentes à des concentrations beaucoup plus faibles. D'autre part les résidus de thrombocytes, retenus sur le filtre, ne contaminent pas le produite final.
Rendement supérieur : notamment en β-thromboglobuline et en "transforming growth factor β", du fait d'un temps de manipulation plus court et de l'absence de centrifugations qui engendrent une dégranulation partielle des thrombocytes.
Meilleure récupération du plasma : au moins 85 % du plasma de départ peuvent être récupérés stérilement et utilisés à d'autres fins.
Possibilité d'utiliser directement du sang total ou des sous fractions telles que les concentrés leuco-plaquettaires, grâce aux filtres retenant spécifiquement les thrombocytes et ne retenant pas les erythrocytes.

L'ensemble de ces avantages confèrent au dispositif un caractère particulièrement avantageux dans une mise en oeuvre d'une préparation de surnageant de thrombocytes activés.

Les applications du produit obtenu grâce à l'invention sont celles d'un surnageant plaquettaire, par exemple :
- La purification de molécules d'origine thrombocytaire ("transforming growth factor β", "platelet-derived growth factor" etc...), à des fins de recherche, de fabrication de réactifs, de fabrication de principes actifs à usage thérapeutique ou autre ;
- La fabrication de préparations à usage thérapeutique ou cosmétologique, et en particulier la préparation d'adjuvants de la réparation tissulaire, par exemple de la cicatrisation cutanée ;
- La réalisation d'un test analytique à usage diagnostique ou prognostique visant à évaluer le contenu des granules thrombocytaires d'un individu.

**TABLEAU I**

| Résultats des analyses effectuées sur les surnageants de thrombocytes activés obtenus lors des manipulations décrites dans les exemples 1 à 6 et moyenne des résultats obtenus lors d'une série de dix manipulations réalisées suivant le protocole décrit dans la demande de brevet WO 86/03122 | | | | | |
|---|---|---|---|---|---|
| | Protéines(1) µg/10⁹ Thr*. | β-TG(2) µg/10⁹ Thr. | PDGF(3) ng/10⁹ Thr. | TGF-β µg/10⁹ Thr. | Activité mitogène /10⁹ Thr. |
| Exemple 1 | 75 | 67 | 17,4 | 1,6 | 44 |
| Exemple 2 | 151 | 60,4 | 11 | 1,7 | 32,2 |
| Exemple 3 | 2160 | 68,8 | 15 | 2 | 48 |
| Exemple 4 | 138 | 20 | 10,6 | 0,8 | 18 |
| Exemple 5 | 62,4 | 11 | 7,6 | 1,1 | 17,2 |
| Exemple 6 | 742 | 9,2 | 8,1 | 2,9 | 5,1 |
| Selon WO 86/03122 | 219 | 38 | 39 | 1,4 | 34 |

| | | | | | |
|---|---|---|---|---|---|
| * Thrombocytes | | | | | |
| (1) Déterminé par la méthode de Bradford (Réactif Biorad réf. 500-0006) | | | | | |
| (2) β-thromboglobuline, déterminé par dosage ELISA (Stago réf. 0419) | | | | | |
| (3) "Platelet-derived growth factor", déterminé par un dosage ELISA | | | | | |
| (4) "Transforming growth factor β", déterminé après chauffage à 60°C pendant 10 heures par la technique de clonage en Agar (Assoian et al., J. Biol Chem, 258, 7555 (1983)) | | | | | |
| (5) La valeur de l'activité mitogène correspond à la dilution de l'échantillon qui induit, après 24 heures de stimulation, la moitié de l'incorporation maximale de thymidine tritiée par des cellules 3T3 clone A 31 à confluence | | | | | |

## Revendications

1. Procédé d'obtention d'une solution de facteurs plaquettaires comportant une étape d'activation de thrombocytes par mise en contact avec une solution d'activateur de thrombocytes, et dans lequel on recueille une solution des facteurs plaquettaires libérés par les thrombocytes lors de l'étape d'activation, caractérisé par le fait que l'on fait passer un liquide contenant une suspension de thrombocytes sur un filtre capable de retenir les thrombocytes, que l'on ajoute une solution d'activateur aux thrombocytes retenus sur le filtre, et que l'on sépare par filtration un filtrat contenant les facteurs plaquettaires en solution tandis que les thrombocytes restent retenus sur le filtre.

2. Procédé selon la revendication 1, caractérisé par le fait qu'avant d'ajouter la solution d'activateur, on ajoute aux thrombocytes retenus sur le filtre une solution de lavage appropriée, et que l'on élimine la solution de lavage par filtration.

3. Procédé selon la revendication 1 ou 2, caractérisé par le fait que l'on utilise comme filtre un système de filtration en profondeur.

4. Procédé selon la revendication 1 ou 2, caractérisé par le fait que l'on utilise comme filtre un système de filtration microporeux fait de fibres creuses.

## Claims

1. Process for obtaining a solution of platelet factors comprising a stage of thrombocyte activation by bringing into contact with a thrombocyte activator solution, and in which a solution of platelet factors released by the thrombocytes during the activation stage is collected, characterized in that a liquid containing a suspension of thrombocytes is passed through a filter capable of retaining the thrombocytes, in that an activator solution is added to the thrombocytes retained on the filter and in that a filtrate containing the platelet factors in solution is separated by filtration whereas the thrombocytes remain retained on the filter.

2. Process according to Claim 1, characterized in that, before adding the activator solution, an appropriate washing solution is added to the thrombocytes retained on the filter and in that the washing solution is removed by filtration.

3. Process according to Claim 1 or 2, characterized in that a deep-filtration system is used as filter.

4. Process according to Claim 1 or 2, characterized in that a microporous filtration system made of hollow fibres is used as filter.

## Patentansprüche

1. Verfahren zum Erhalt einer Lösung von Plättchen-Faktoren, das einen Schritt der Aktivierung von Thrombozyten durch In-Kontakt-Bringen mit einer Thrombozytenaktivator-Lösung umfaßt und in dem man eine Lösung von Plättchen-Faktoren, die von den Thrombozyten während des Aktivierungsschritts freigesetzt werden, gewinnt, dadurch gekennzeichnet, daß man
- eine Flüssigkeit, die eine Suspension von Thrombozyten enthält, durch ein Filter laufen läßt, das die Thrombozyten zurückhalten kann;
- den auf dem Filter zurückgehaltenen Thrombozyten eine Aktivator-Lösung zusetzt; und
- durch Filtration ein Filtrat abtrennt, das die Plättchen-Faktoren in Lösung enthält, während die Thrombozyten auf dem Filter zurückbleiben.

2. Verfahren nach Anspruch 1, dadurch gekennzeichnet, daß man vor dem Zusatz der Aktivator-Lösung den auf dem Filter zurückgehaltenen Thrombozyten eine geeignete Waschlösung zusetzt und daß man die Waschlösung durch Filtration entfernt.

3. Verfahren nach Anspruch 1 oder Anspruch 2, dadurch gekennzeichnet, daß man als Filter ein Tiefen-Filtrationssystem verwendet.

4. Verfahren nach Anspruch 1 oder Anspruch 2, dadurch gekennzeichnet, daß man als Filter ein aus Hohlfasern hergestelltes Mikroporen-Filtrationssystem verwendet.
